(19) **Europäisches Patentamt / European Patent Office / Office européen des brevets**

(11) **EP 4 289 358 B1**

(12) **EUROPEAN PATENT SPECIFICATION**

(45) Date of publication and mention of the grant of the patent:
**11.12.2024   Bulletin 2024/50**

(21) Application number: **23178202.0**

(22) Date of filing: **08.06.2023**

(51) International Patent Classification (IPC):
*A61B 5/346* (2021.01)    *A61B 5/349* (2021.01)
*A61B 5/00* (2006.01)    *A61B 5/021* (2006.01)
*A61B 5/02* (2006.01)    *A61B 5/36* (2021.01)
*A61B 5/358* (2021.01)    *A61B 5/357* (2021.01)
*A61B 5/355* (2021.01)    *A61B 5/353* (2021.01)
*A61B 5/352* (2021.01)

(52) Cooperative Patent Classification (CPC):
**A61B 5/7278; A61B 5/021; A61B 5/346**

(54) **ESTIMATING BLOOD PRESSURE OF A SUBJECT USING AN ECG DRIVEN CARDIOVASCULAR MODEL**

SCHÄTZUNG DES BLUTDRUCKS EINER PERSON UNTER VERWENDUNG EINES EKG-GESTEUERTEN KARDIOVASKULÄREN MODELLS

ESTIMATION DE LA PRESSION SANGUINE D'UN SUJET À L'AIDE D'UN MODÈLE CARDIOVASCULAIRE PILOTÉ PAR ECG

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **10.06.2022   IN 202221033450**

(43) Date of publication of application:
**13.12.2023   Bulletin 2023/50**

(73) Proprietor: **Tata Consultancy Services Limited Maharashtra (IN)**

(72) Inventors:
- **ROY, Dibyendu**
  **700160 Kolkata, West Bengal (IN)**
- **MAZUMDER, Oishee**
  **700160 Kolkata, West Bengal (IN)**
- **SINHA, Aniruddha**
  **700160 Kolkata, West Bengal (IN)**
- **KHANDELWAL, Sundeep**
  **201309 Noida, Uttar Pradesh (IN)**
- **GHOSE, Avik**
  **700160 Kolkata, West Bengal (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
- **ROY DIBYENDU ROY DIBYENDU@TCS COM ET AL: "Wearable sensor driven Cardiac model to derive hemodynamic insights during exercise", PROCEEDINGS OF THE 1ST WORKSHOP ON SECURITY AND PRIVACY FOR MOBILE AI, ACMPUB27, NEW YORK, NY, USA, 24 June 2021 (2021-06-24), pages 30 - 35, XP058566843, ISBN: 978-1-4503-7357-9, DOI: 10.1145/3469260.3469670**
- **SILVA LUCAS RAPHAEL BENTO ET AL: "Exponential model for analysis of heart rate responses and autonomic cardiac modulation during different intensities of physical exercise", ROYAL SOCIETY OPEN SCIENCE, vol. 6, no. 10, 16 October 2019 (2019-10-16), pages 190639, XP093079233, DOI: 10.1098/rsos.190639**

**Description**

CROSS-REFERENCE TO RELATED APPLICATIONS AND PRIORITY

The present application claims priority to Indian application, Application No. 202221033450, filed in India on 10th June 2022.

TECHNICAL FIELD

The disclosure herein generally relates to the field of in-silico modeling of hemodynamic patterns of physiologic blood flow, and, more particularly, to systems and methods for estimating blood pressure of a subject using an electrocardiogram (ECG) driven cardiovascular model.

BACKGROUND

Computer simulation-based cardiovascular modeling in healthcare is an attractive proposition since analytical models aid in improving understanding of cardiac physiology which in turn is useful for predicting adverse accidents like sudden cardiac death. Clinicians find predictive models useful to stratify likelihood or severity of exercise intolerance in patients. In-silico model platforms also serve as virtual test-beds to verify consequences on different levels of exercise for pathological conditions of varying severity.

Various literatures define cardiac parameter variations based on lumped order models. Conventional cardiovascular hemodynamic models depend on neuromodulation schemes (baroreflex autoregulation) and threshold parameters of neuromodulation correlate with physical activities. Thus these models may not work practically for a large set of people due to dependency on prior knowledge of these parameters. Establishing a cardiac care continuum for cardiac rehabilitation may not be as effective as desired. Document ROY DIBYENDU ET AL., in "Wearable sensor driven Cardiac model to derive hemodynamic insights during exercise", discloses a cardiovascular digital twin platform to simulate the effect of exercise on various cardiac parameters of medical importance. The model incorporates the real-time ECG signal from the body-worn sensors to estimate exercise level and compute cardiac variables like left ventricular dynamics, cardiac output, ejection fraction, mean arterial pressure, etc., of an individual while performing exercises. This document determines the cardiac compliances from the morphology of the single-lead ECG signal and Systemic resistance estimation from the information of the exercise level for a specific subject performing physical activities. Simulation results are produced employing an open-source database (Troika). This document proposed framework that can aid to progressively track the cardiovascular efficiency during exercise for patients with cardiac co-morbidity and act as a personalized therapy guide in a care continuum scenario (Abstract). Document SILVA LUCAS RAPHAEL BENTO ET AL in "Exponential model for analysis of heart rate responses and autonomic cardiac modulation during different intensities of physical exercise" discloses to compare the heart rate (HR) dynamics and variability before and after high-intensity interval training (HIIT) and moderate-intensity continuous training (MICT) protocols with workloads based on treadmill workload at which maximal oxygen uptake was achieved ($WLVO_2max$). Ten participants performed cardiopulmonary exercise testing (CPET) to obtain oxygen uptake ($WLVO_2max$). All training protocols were performed on a treadmill, with 0% grade, and had similar total distance. The MICT was composed b 21 mins at 70% of $WLVO_2max$. The first HIIT protocol (HIIT-30: 30) was composed by 29 repetitions of 30 s at 100% of $SVO_2max$ and the second HIIT protocol (HIIT-4: 3) was composed by three repetitions of 4 min at 90% of $WLVO_2max$. Before, during and after each training protocol, HR dynamics and variability (HRV) were analysed by standard kinetics and linear (time and frequency domains). The repeated measures analysis of variance indicated that the HR dynamics, which characterizes the speed of HR during the rest to exercise transition, was statistically ($p < 0.05$) slower during MICT in comparison to both HIIT protocols. The HRV analysis, which characterizes the cardiac autonomic modulation during the exercise recovery, was statistically higher in HIIT-4: 3 in comparison to MICT and HIIT-30: 30 protocols ($p < 0.005$ and $p = 0.012$, respectively), suggesting that the HIIT-4: 3 induced higher sympathetic and lower parasympathetic modulation during exercise in comparison to the other training protocols. In conclusion, HIIT-4: 3 demonstrated post-exercise sympathetic hyperactivity and a higher HR peak, while the HIIT-30: 30 and MICT resulted in better HRV and HR in the exercise-recovery transition. The cardiac autonomic balance increased in HIIT-30: 30 while HIIT-4: 3 induced sympathetic hyperactivity and cardiac overload (Abstract).

SUMMARY

Embodiments of the present disclosure present technological improvements as solutions to one or more of the above-mentioned technical problems recognized by the inventors in conventional systems.

In an aspect, there is provided a processor implemented method comprising estimating, by an in-silico cardiovascular hemodynamic model via one or more hardware processors, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on: (i) a set of PQRST amplitudes; and (ii) time-instances, (

$$\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]_{; j \in m}$$

for a $j^{th}$ cardiac cycle ($\forall j \in m$) of the ECG signal; generating, by the in-silico cardiovascular hemodynamic model via the one or more hardware processors, a set of compliance functions using the estimated cardiac parameters; sequentially activating, by the in-silico cardiovascular hemodynamic model via the one or more hardware processors, a plurality of cardiac chambers, in a synchronized manner, using the generated set of compliance functions; and estimating blood pressure of the subject, by the in-silico cardiovascular hemodynamic model via the one or more hardware processors, wherein the in-silico cardiovascular hemodynamic model is driven by the ECG signal associated with the subj ect.

In another aspect, there is provided a system comprising a memory storing instructions in an in-silico cardiovascular hemodynamic model; one or more communication interfaces; and one or more hardware processors coupled to the memory via the one or more communication interfaces, wherein the one or more hardware processors are configured by the instructions to: estimate, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on: (i) a set of PQRST amplitudes; and (ii) time-instances, (

$$\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]_{; j \in}$$

$m$) for a $j^{th}$ cardiac cycle (Vj E $m$) of the ECG signal; generate, a set of compliance functions using the estimated cardiac parameters; sequentially activate, a plurality of cardiac chambers, in a synchronized manner, using the generated set of compliance functions; and estimate blood pressure of the subject, by the in-silico cardiovascular hemodynamic model via the one or more hardware processors, wherein the in-silico cardiovascular hemodynamic model is driven by the ECG signal associated with the subject.

In yet another aspect, there are provided one or more non-transitory machine-readable information storage mediums comprising one or more instructions which when executed by one or more hardware processors cause estimating, by an in-silico cardiovascular hemodynamic model via one or more hardware processors, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on: (i) a set of PQRST amplitudes; and (ii) time-instances,

$$\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$$

$_($ $]; j \in m$) for a $j^{th}$ cardiac cycle ($\forall j \in m$) of the ECG signal; generating, by the in-silico cardiovascular hemodynamic model via the one or more hardware processors, a set of compliance functions using the estimated cardiac parameters; sequentially activating, by the in-silico cardiovascular hemodynamic model via the one or more hardware processors, a plurality of cardiac chambers, in a synchronized manner, using the generated set of compliance functions; and estimating blood pressure of the subject, by the in-silico cardiovascular hemodynamic model via the one or more hardware processors, wherein the in-silico cardiovascular hemodynamic model is driven by the ECG signal associated with the subj ect.

In the present invention, the one or more hardware processors are configured to estimate HR based on the HR associated with a noise-less ECG signal, when the ECG signal is missing or is noisy, and is represented as:

$$HR(t) = \begin{cases} h_{ae}(end) + w(t) & during\ resting\ state \\ \left[1 - (t+1)e^{-\frac{t}{\tau_k}}\right] h_{ae}(0) + w(t) & during\ exercising\ state \end{cases}$$

where, $h_{ae}$ (end), $h_{ae}$ (0) are the HRs at a last and a first instance of capturing the ECG signal respectively, w($t$) = H(0, $\sigma^2$) is white-noise with zero-mean, variance of $\sigma^2$ = 9.26, and $\tau_k$ = 50 sec defines the time constant, $\sigma^2$, and $\tau_k$ are learnt empirically through the ECG signal using linear regression.

It is to be understood that both the foregoing general description and the following detailed description are exemplary and explanatory only and are not restrictive of the invention, as claimed. The invention is defined in the claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG.1 illustrates an exemplary block diagram of a system for estimating blood pressure of a subject using an electrocardiogram (ECG) driven cardiovascular hemodynamic model, in accordance with some embodiments of the present disclosure.

FIG.2 illustrates an exemplary block diagram of the cardiovascular hemodynamic model, in accordance with some embodiments of the present disclosure.

FIG.3 illustrates an exemplary flow diagram of a computer implemented method for estimating blood pressure of a subject using an electrocardiogram (ECG) driven cardiovascular model, in accordance with some embodiments of the present disclosure.

FIG. 4A illustrates an ECG signal with PQRST amplitudes and time steps, in accordance with some embodiments of the present disclosure.

FIG. 4B illustrates synchronous activation signals to trigger cardiac chambers of an in-silico ECG driven cardiovascular hemodynamic model, in accordance with some embodiments of the present disclosure.

FIG.5 illustrates an estimated heart-rate of a subject 1 of the Kaggle dataset, in accordance with some embodiments of the present disclosure.

FIG. 6A illustrates a force-sensing-resistor (FSR) signal, as known in the art.

FIG. 6B illustrates a simulated blood pressure of the subject 1 with respect to the FSR signal of FIG.6A, in accordance with some embodiments of the present disclosure.

FIG.7 illustrates a correlation between ground-truth blood pressure and measured blood pressure of all the subjects of Kaggle dataset in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Cardiovascular diseases (CVD) are a main cause of death worldwide; with coronary heart disease (CHD) accounting for a majority of CVD mortality. CHD has a high prevalence and is aggravated by lifestyle disorders. Exercise-based cardiac rehabilitation is often prescribed as a prevention scheme to reduce the impact of CHD. Cardiac rehabilitation (CR) is a complex secondary preventive intervention that aims to optimize cardiovascular disease risk reduction, promoting adoption and adherence of healthy habits, and reducing disability among those with established CHD. CR is prescribed to patients suffering from cardiac diseases like valvular heart disease, heart transplantation, heart failure with reduced ejection fraction (EF), post-coronary artery bypass grafting (CABG), etc. with the goal of improving quality of life and reducing re-hospitalization. Although CR is a multi-component risk management process, exercise is considered as an integral component. Exercise has been shown to regulate several established CHD risk factors like blood pressure, blood lipid profile, glucose metabolism, weight status and body composition through cardiovascular and metabolic adaptation. In-silico models serve as virtual test beds to verify consequences on different levels of exercise for pathological conditions of varying severity.

Machine learning based conventional cardiovascular hemodynamic models are dependent on prior knowledge of threshold parameters of neuromodulation schemes, thereby limiting their application in establishing a cardiac care continuum for cardiac rehabilitation. Applicants' previous patent application No. 202121010972 provided compliance functions for activating cardiac chambers of cardiovascular hemodynamic models, however, the parameters used were constants. The present disclosure enables estimating cardiac parameters from an electrocardiogram (ECG) signal associated with a subject using the morphology of the ECG signal, thereby reproducing activation delays in the cardiac chambers purposefully. In accordance with the present disclosure, the blood pressure of the subject is also estimated using the ECG signal even if the signal is missed for some time instance(s) or is noisy.

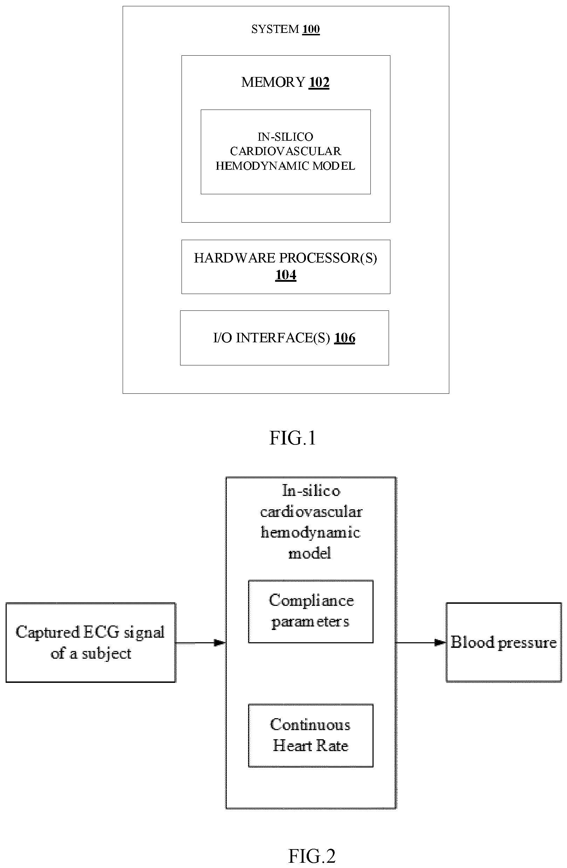

Referring now to the drawings, and more particularly to FIG. 1 through FIG. 7, where similar reference characters denote corresponding features consistently throughout the figures, there are shown preferred embodiments and these embodiments are described in the context of the following exemplary system and/or method.

Based on the requirement of both exercise monitoring and in-silico modeling for establishing a cardiac care continuum for cardiac rehabilitation, the present disclosure provides cardiovascular digital-twin simulation system as shown in FIG. 1 that illustrates an exemplary block diagram of a system 100 for estimating cardiac parameters when performing an activity using a personalized cardiovascular hemodynamic model, in accordance with some embodiments of the present disclosure. In an embodiment, the system 100 includes one or more hardware processors 104, communication interface device(s) or input/output (I/O) interface(s) 106, and one or more data storage devices or memory 102 operatively coupled to the one or more hardware processors 104. The one or more hardware processors 104 can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In the context of the present disclosure, the expressions 'processors' and 'hardware processors' may be used interchangeably. In an embodiment, the system 100 can be implemented in a variety of computing systems, such as laptop computers, notebooks, hand-held devices, workstations, mainframe computers, servers, a network cloud and the like.

I/O interface(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface(s) can include one or more ports for connecting a number of devices to one another or to another server.

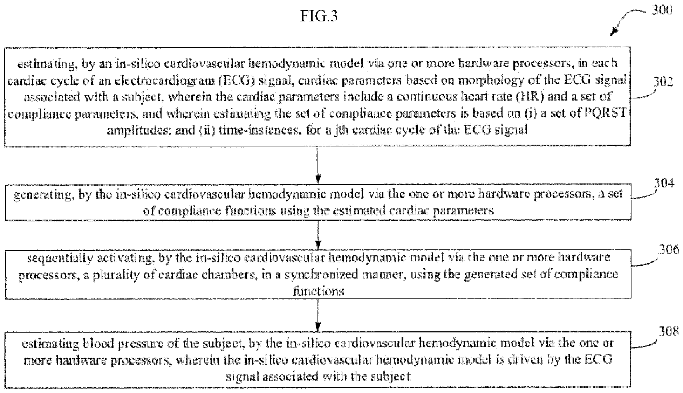

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, one or more modules (e.g. the in-silico cardiovascular hemodynamic model) of the system 100 can be stored in the memory 102. FIG.2 illustrates an exemplary block diagram of a cardiovascular hemodynamic model, in accordance with some embodiments of the present disclosure while FIG.3 illustrates an exemplary flow diagram of a computer implemented method 300 for estimating blood pressure of a subject using an electrocardiogram (ECG) driven cardiovascular model, in accordance with some embodiments of the present disclosure. In an embodiment, the system 100 includes one or more data storage devices or memory 102 operatively coupled to the one or more hardware processors 104 and is configured to store instructions configured for execution of steps of the method 200 by the one or more hardware processors 104. The memory 102 further comprises (or may further comprise) information pertaining to input(s)/output(s) of each step performed by the system and method of the present disclosure. In other words, input(s) fed at each step and output(s) generated at each step are comprised in the memory 102 and can be utilized in further processing and analysis, particularly functionalities represented by modules (the in-silico cardiovascular hemodynamic model) illustrated in FIG. 1 and FIG.2. The modules are implemented as at least one of a logically self-contained part of a software program, a self-contained hardware component, and/or, a self-contained hardware component with a logically self-contained part of a software program embedded into each of the hardware component that when executed perform the method 300 described hereinafter. Accordingly, the modules are invoked by the one or more hardware processors 104 to perform the method 300 of the present disclosure.

The steps of the method 300 will now be explained in detail with reference to the components of the system 100 of FIG. 1 and the block diagram of FIG.2. Although process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

The human cardiovascular system contains a couple of atrium and ventricles acting like a pulsatile pump. The systemic circulation is produced by the left-ventricle (*lv*) and left-atrium (*lv*) pumping oxygenated blood to all body tissues via aorta. On the other side, the right-ventricle *(rv)* and right-atrium *(ra)* drive deoxygenated blood to the lungs forming the pulmonic circulation. The rhythmic unidirectional blood flow across the cardiac chambers is controlled by four heart valves, namely, mitral (*mi*) and aortic (*ao*) valves in the left heart and tricuspid (*tr*) and pulmonic (*pu*) valves in the right heart respectively which are synchronously opened and closed based on the pressure difference across the chambers. Hence, the hemodynamics of the human circulatory system outlines the dynamics of blood flow as a function of pressure-volume fluctuations across cardiac chambers. Additionally, the homeostatic process of autoregulation continuously monitors and regulates blood flow throughout the body. So, to get the quantitative overview of a cardiac system, it is necessary to monitor the hemodynamics of the cardiac chambers such as pressures, volumes, blood-flows, etc.

According to the electrophysiology principle, each of the heart chambers is simultaneously actuated by an autonomous compliance function. During each cardiac cycle, the activation begins in the sinoatrial node located inside the right atrium (*ra*), then spreads throughout the atrium (depolarization of atria). It then propagates to the ventricles after passing through the atrioventricular node, bundle of His and the Purkinje fibers (depolarization and repolarization of ventricles). As a consequence, during the repolarization state, the ventricles fill with incoming blood from atrium, and in the depolarization state, blood ejects from ventricles. The autonomous activation functions across the cardiac chambers can analytically be defined as compliance functions given below.

$$u_{ra}(t) = \begin{cases} 0, & 0 \le t \le T_a \\ 1 - \cos\left(2\pi \frac{t-T_a}{T-T_a}\right), & T_a \le t < T \end{cases} \tag{1}$$

$$u_{la}(t) = \begin{cases} 0, & d_{la} \le t \le (T_a + d_{la}) \\ 1 - \cos\left(2\pi \frac{t-T_a}{T-T_a}\right), & (T_a + d_{la}) \le t < T \end{cases} \tag{2}$$

$$u_i(t) = \begin{cases} 1 - \cos\left(\pi \frac{t}{T_1}\right), & d \le t \le (T_1 + d) \\ 1 + \cos\left(\pi \frac{t-T_1}{T_2-T_1}\right), & (T_1 + d) \le t \le (T_2 + d) \\ 0, & (T_2 + d) \le t < T \end{cases} \tag{3}$$

where, $i \in \{lv, rv\}$, time t is considered over a complete cardiac cycle ($T$), $T_a$, ($T_a + d_{la}$) are the activation times across ($ra$) and ($la$) respectively, and ($T_1 + d$), ($T_2 + d$) define the systolic and diastolic activation times across the ventricles.

Thus, the compliance action of the cardiac chambers is represented as:

$$C_{ra}(t) = C_{ra}^{min} + 0.5 \times \left(C_{ra}^{max} - C_{ra}^{min}\right)u_{ra}(t) \tag{4}$$

$$C_{la}(t) = C_{la}^{min} + 0.5 \times \left(C_{la}^{max} - C_{la}^{min}\right)u_{la}(t) \tag{5}$$

$$C_i(t) = C_i \times u_i(t), \quad i \in \{lv, rv\} \tag{6}$$

where, $C_{ra}^{min}, C_{la}^{min}$ are the minimum values, and $C_{ra}^{max}, C_{la}^{max}$ are the maximum values of the ($ra$) and ($la$) compliance functions respectively,
$C_i$; Vi E {$lv, rv$} is the systolic compliance across ($lv$) and ($rv$).

Now, considering equations (1) - (3), it is observed that there are several dynamic parameters (such as heart-rate ($T$), ($ra$) activation time ($T_a$), ($la$) activation time ($T_a + d_{la}$), ventricular systolic ($T_1 + d$), and diastolic ($T_2 + d$) times. As mentioned above, in Applicants previous patent application No. 202121010972, these parameters were considered as constants. In accordance with the present disclosure, these parameters are estimated in each cardiac cycle of the ECG signal associated with the subject to simulate the in-silico ECG driven cardiovascular hemodynamic model in line with the human cardiovascular system as explained above. It may be noted that in the context of the present disclosure, the expressions "in-silico cardiovascular hemodynamic model", "cardiovascular model" and "cardiovascular hemodynamic model" may be used interchangeably.

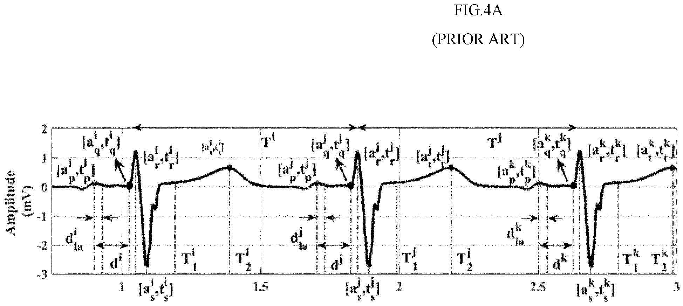

Accordingly, in an embodiment of the present disclosure, an in-silico cardiovascular hemodynamic model via the one or more hardware processors 104, is configured to estimate, at step 302, cardiac parameters based on morphology of an electrocardiogram (ECG) signal associated with a subject in each cardiac cycle of the ECG signal. The ECG signal may be captured using sensors in say a wearable device, such as a digital watch. The cardiac parameters include a continuous heart rate (HR) and a set of compliance parameters for each cardiac cycle. FIG.4A illustrates an ECG signal with PQRST amplitudes and time steps, as known in the art.

In accordance with the present disclosure, to estimate the continuous HR of the ECG signal, the R-to-R interval of each cardiac cycle is identified as shown in FIG. 4A. Let us assume that $D = [t^1, t^2, \dots, t^m]$; $m \in \mathbb{R}^+$ defines the set containing the durations of the cardiac cycles of the entire ECG signal. Thus, the continuous heart-rate (HR) of the ECG data is $HR = 60/D$.

In accordance with the present disclosure, the compliance parameters are estimated based on:

(i) a set of PQRST amplitudes; and

(ii) time-instances, ( $\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$ ; $j \in d$) for a $j^{th}$ cardiac cycle ($\forall j \in m$) of

the ECG signal (refer FIG. 4A).

It is often observed that the ECG signal during physical activities (such as exercise) is very noisy. Furthermore, in some window, the ECG signal may be missing in scenarios where the sensor may be disconnected at least momentarily. In such scenarios, the present disclosure enables estimating an approximate HR based on the HR information as measured through noise-less ECG signal. In accordance with an embodiment of the present disclosure, the continuous HR estimation is represented as:

$$HR(t) = \begin{cases} h_{ae}(end) + w(t) & during\ resting\ state \\ \left[1 - (t+1)e^{-\frac{t}{\tau_k}}\right]h_{ae}(0) + w(t) & during\ exercising\ state \end{cases} \quad (7)$$

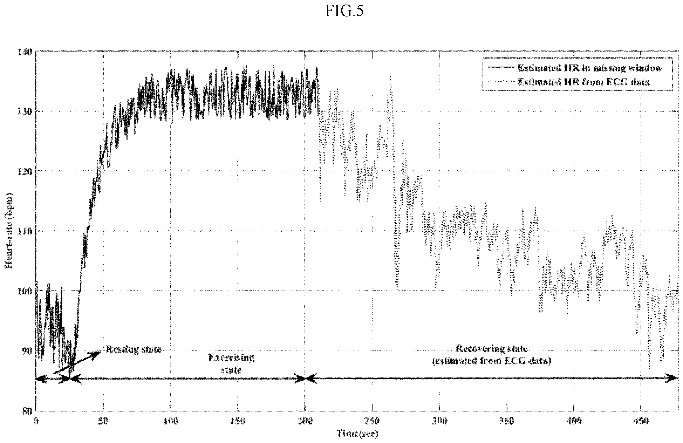

where, $h_{ae}$ (end), $h_{ae}$ (0) are the HRs at a last and a first instance of capturing the ECG signal respectively, $w(t)$ = H(0, $\sigma^2$) is white-noise with zero-mean, variance of $\sigma^2$ = 9.26, and $\tau_k$ = 50 sec defines the time constant, $\sigma^2$, and $\tau_k$ are learnt empirically through the ECG signal using linear regression. FIG.5 illustrates an estimated heart-rate of a subject 1 of Kaggle dataset (explained later in the description), in accordance with some embodiments of the present disclosure, wherein solid line represents an estimated HR in a missing window (ECG signal is missing) and dotted line represents an estimated HR from the ECG signal. From FIG. 5, it is perceived that the HR is within a normal HR range (90-100 bpm) in a resting state and it starts increasing during an exercising state. Once, the physical activities are completed, it again starts decreasing and approaching towards the normal HR range.

In an embodiment, the set of compliance parameters of the $j^{th}$ cardiac cycle is estimated as:

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \quad T^j = t^j, \quad (8)$$

wherein,

$T_a^j$ is the activation time across a right atrium (ra),

time $t$ is considered over a complete cardiac cycle,

$d_{la}$ is a time delay between activation of the ra and a left atrium la,

$d$ is a time delay when activation of a plurality of cardiac chambers traverses from an atrium to ventricles therein,

$C_{ra}^{max,j}$ and $C_{ra}^{min,j}$ represent a maximum and a minimum value of ra compliance,

$C_i$ represents compliance functions to actuate a right ventricle rv and a left ventricle lv, and

$g_1$ = 5, $g_2$ = $g_3$ = 2, and $g_4$ = 1 are set empirically.

Accordingly, in accordance with the present disclosure, the in-silico cardiovascular hemodynamic model via the one or more hardware processors 104, is configured to generate, at step 304, a set of compliance functions using the estimated cardiac parameters as represented by equations (4-6) above.

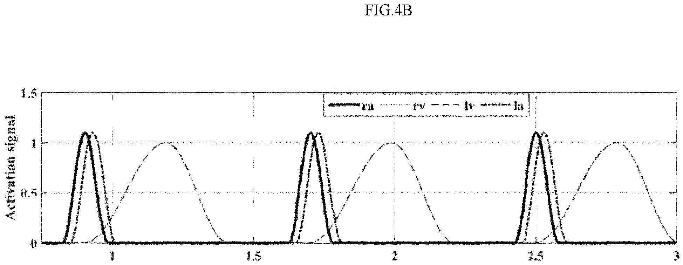

The cardiac chambers are activated sequentially, in a synchronized manner, by the time-varying compliance functions explained above. Typically, this activation starts from the sinoatrial node, which is located inside *ra,* then, it traverses to the *la* with a time delay of $d_{la}$, causing them to contract for pumping the blood into the ventricles. After that, the activation traverses from the atrium to the ventricles via an atrioventricular node with a time delay of d, allowing the ventricles to fill with blood. In accordance with the present disclosure, the in-silico cardiovascular hemodynamic model via the one or more hardware processors 104, is configured to sequentially activate, at step 306, the cardiac chambers of the in-silico ECG driven cardiovascular hemodynamic model, in a synchronized manner, using the generated set of compliance functions. FIG. 4B illustrates synchronous activation signals to trigger cardiac chambers of an in-silico ECG driven cardiovascular hemodynamic model, in accordance with some embodiments of the present disclosure.

In accordance with the present disclosure, the in-silico cardiovascular hemodynamic model via the one or more hardware processors is configured to estimate blood pressure of the subject, at step 308, wherein the in-silico cardiovascular hemodynamic model is driven by the ECG signal associated with the subject.

SIMULATION RESULTS

A study was performed using Kaggle dataset (provided in "Nonlinear cuffless blood pressure estimation of healthy subjects using pulse transit time and arrival time", - IEEE Transactions on Instrumentation and Measurement by A.Esmaili et al.) containing ECG data (signal) for 32 subjects (age range of 21-50 years) while exercising on a treadmill. The meta-data information (such as age, height, and weight) is also provided. In this experiment, all subjects were requested to run on a treadmill for 3 min at 8km/hr speed. After completing the exercise, the data collection started immediately. The whole data collection process consumed around 4-5 min per subject. Additionally, during data-collection, several reference Blood Pressure (BP) are measured by a traditional BP measurement technique.

As the data-collection step begins immediately after the exercise is over, so, the ECG data during the exercise is missing. To overcome this problem, it was assumed that each subject was initially in a resting state for 25 sec, then, performs the treadmill work out for 3 min. Hence, for this specific time-frame, the heart-rate (HR) needs to be estimated to simulate the cardiovascular hemodynamic model based on equation (7) above.

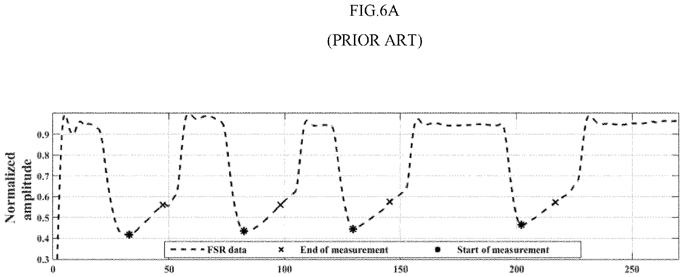

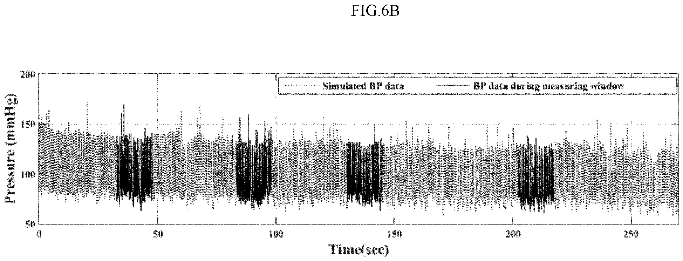

For validating responses of the in-silico ECG driven cardiovascular hemodynamic model of the present disclosure, ground truth BP of the Kaggle dataset was utilized. This data contains force-sensing-resistor (FSR) signal for determining the BP estimation time-instances. FIG. 6A illustrates a force-sensing-resistor (FSR) signal, as known in the art while FIG. 6B illustrates a simulated blood pressure of the subject 1 with respect to the FSR signal of FIG.6A, in accordance with some embodiments of the present disclosure. Table 1A and Table 1B below shows a comparative study between the ground truth BP versus simulated BP of various subjects of the Kaggle dataset.

Table 1A:

| Subject ID | Age | BMI (kg/m2) | | SBP (mmHg) | | | |
|---|---|---|---|---|---|---|---|
| | | | | 1st window | 2nd window | 3rd window | 4th window |
| 1 | 25 | 21.22 | Ground Truth | 137 | 134 | 125 | 122 |
| | | | Simulated | 136.95 | 133.9 | 125.41 | 121.88 |
| 6 | 50 | 32.87 | Ground Truth | 165 | 161 | 143 | 134 |
| | | | Simulated | 165.5 | 160.9 | 143.7 | 132.9 |
| 19 | 27 | 29.4 | Ground Truth | 151 | 148 | 143 | 135 |
| | | | Simulated | 150.95 | 147.9 | 142.97 | 135.26 |

Table 1B:

| Subject ID | Age | BMI (kg/m2) | | DBP (mmHg) | | | |
|---|---|---|---|---|---|---|---|
| | | | | 1st window | 2nd window | 3rd window | 4th window |
| 1 | 25 | 21.22 | Ground Truth | 78 | 75 | 71 | 67 |
| | | | Simulated | 78.07 | 74.21 | 71.17 | 65.6 |

(continued)

| Subject ID | Age | BMI (kg/m2) | | DBP (mmHg) | | | |
|---|---|---|---|---|---|---|---|
| | | | | 1st window | 2nd window | 3rd window | 4th window |
| 6 | 50 | 32.87 | Ground Truth | 89 | 83 | 82 | 81 |
| | | | Simulated | 89.6 | 84.2 | 82.2 | 82 |
| 19 | 27 | 29.4 | Ground Truth | 80 | 81 | 77 | 79 |
| | | | Simulated | 80.3 | 81.26 | 76.8 | 79.4 |

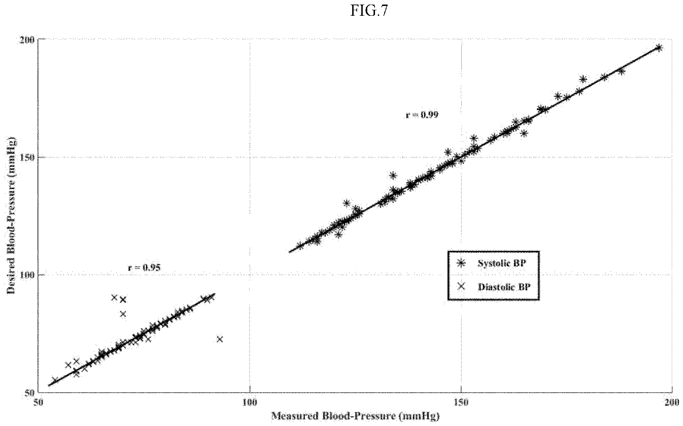

On comparing the ground-truth and simulated BPs (Table 1A and 1B) of different subjects (of varying age and BMI), it is seen that the simulated systolic BP (SPB) and diastolic BP (DBP) are close to the ground truth BP. Additionally, the correlation between the ground-truth BP [systolic (SBP) and diastolic (DBP)] and the measured BP of all the subjects is shown in FIG.7.

In developing countries, coronary heart disease (CHD) is a leading cause of death. Lifestyle disorders are one of the precursors of CHD. Exercise-based cardiac health rehabilitation is often prescribed as a secondary prevention strategy to reduce the impact of CHD. The present disclosure proves an in-silico cardiac platform to simulate the exercise effect on cardiac parameters relevant for cardiac rehabilitation. The method and system of the present disclosure enable simulating an individual's (subject) exercise condition using the ECG signal approximated from say a wearable device. Neuromodulation parameters are estimated from the morphology of the ECG signal. Effect of exercise on cardiac parameters are simulated on open-source Kaggle dataset for healthy subjects, and hemodynamic parameters are evaluated.

The cardiac parameters estimated from the ECG signal associated with a subject, are integrated into the practical application of estimating blood pressure of the subject using the in-silico ECG driven cardiovascular model and with a meaningful combination of additional elements of generating the set of compliance functions and using the estimated parameters to sequentially activate the cardiac chambers of the in-silico ECG driven cardiovascular hemodynamic model.

As illustrated in FIG.7, the model simulated BP shows a correlation coefficient of 0.99 (for systolic BP), and 0.95 (for diastolic BP) with Kaggle data BP. The method and system of the present disclosure find application as a cardiac rehabilitation monitoring aid for both healthy and patients suffering from heart diseases, thereby providing cardiac care continuum.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (300) comprising:

   estimating, by an in-silico cardiovascular hemodynamic model via one or more hardware processors, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on:

   (i) a set of PQRST amplitudes; and

   (ii) time-instances, ( $\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$ $_{; j \in m}$)

   for a $j^{th}$ cardiac cycle ($\forall j \in m$) of the ECG signal (302),
   wherein the continuous HR is estimated, when the ECG signal is missing or noisy, and is represented as:

   $$HR(t) = \begin{cases} h_{ae}(end) + w(t) & during\ resting\ state \\ \left[1 - (t+1)e^{-\frac{t}{\tau_k}}\right] h_{ae}(0) + w(t) & during\ exercising\ state \end{cases}$$

   where t is time considered over a complete cardiac cycle (T), $h_{ae}$ (end) is the HR at a last instance of capturing of the ECG signal in the resting state and $h_{ae}$ (0) is the HR at a first instance of the capturing of the ECG signal in the exercise state, wherein the subject is initially in the resting state and then performs exercise to reach the exercise state, $w(t) = \mathbb{N}(0, \sigma^2)$ is white-noise with zero-mean, variance of $\sigma^2$ = 9.26, and $\tau_k$ = 50 sec defines a time constant, and wherein the $\sigma^2$, and the $\tau_k$ are learnt empirically through the ECG signal using linear regression;
   generating, by the in-silico cardiovascular hemodynamic model via the one or more hardware processors, a set of compliance functions using the estimated cardiac parameters (304);
   sequentially activating, by the in-silico cardiovascular hemodynamic model via the one or more hardware processors, a plurality of cardiac chambers of the in-silico cardiovascular hemodynamic model, in a synchronized manner, using the generated set of compliance functions (306); and
   estimating blood pressure of the subject, by the in-silico cardiovascular hemodynamic model via the one or more hardware processors, using the estimated cardiac parameters and the sequentially activated plurality of cardiac chambers to simulate the in-silico cardiovascular hemodynamic model in line with a human cardiovascular system of the subject, wherein the in-silico cardiovascular hemodynamic model is driven by the ECG signal associated with the subject (308).

2. The processor implemented method as claimed in claim 1, wherein the set of compliance parameters of the $j^{th}$ cardiac cycle is estimated as

   $$T_a^j = t_p^j,$$

$$d_{la}^{j} = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \ T^j = t^j,$$

wherein,

$T_a^j$ is the activation time across a right atrium (*ra*),

time t is considered over a complete cardiac cycle,

$d_{la}$ is a time delay between activation of the *ra* and a left atrium *la,*

*d* is a time delay when activation of the plurality of cardiac chambers traverses from an atrium to ventricles therein,

$C_{ra}^{max,j}$ and $C_{ra}^{min,j}$ represent a maximum and a minimum value of *ra* compliance,

$C_i$ represents compliance functions to actuate a right ventricle *rv* and a left ventricle *lv*, and

$g_1 = 5$, $g_2 = g_3 = 2$, and $g_4 = 1$ are set empirically.

3. A system (100) comprising:

a memory (102) storing instructions in an in-silico cardiovascular hemodynamic model;
one or more communication interfaces (106); and
one or more hardware processors (104) coupled to the memory (102) via the one or more communication interfaces (106), wherein the one or more hardware processors (104) are configured by the instructions to:

estimate, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on:

(i) a set of PQRST amplitudes; and

(ii) time-instances, $\left( \left[ \left(a_p^j, t_p^j\right), \left(a_q^j, t_q^j\right), \left(a_r^j, t_r^j\right), \left(a_s^j, t_s^j\right), \left(a_t^j, t_t^j\right) \right]_{;j \,\in\, m} \right)$

for a $j^{th}$ cardiac cycle ($\forall j \in m$) of the ECG signal,
wherein the continuous HR is estimated, when the ECG signal is missing or noisy, and is represented as:

$$HR(t) = \begin{cases} h_{ae}(end) + w(t) & during\ resting\ state \\ \left[1 - (t+1)e^{-\frac{t}{\tau_k}}\right] h_{ae}(0) + w(t) & during\ exercising\ state \end{cases}$$

where t is time considered over a complete cardiac cycle (T), $h_{ae}$ (end) is the HR at a last instance of capturing of the ECG signal in the resting state and $h_{ae}$ (0) is the HR at a first instance of the capturing of the ECG signal in the exercise state, wherein the subject is initially in the resting state and then performs exercise to reach the exercise state, w(t) = $\mathbb{N}(0, \sigma^2)$ is white-noise with zero-mean, variance of $\sigma^2$ = 9.26, and $\tau_k$ = 50 sec defines a time constant, and wherein the $\sigma^2$, and the $\tau_k$ are learnt empirically through the ECG signal using linear regression;
generate, a set of compliance functions using the estimated cardiac parameters;
sequentially activate, a plurality of cardiac chambers of the in-silico cardiovascular hemodynamic model, in a synchronized manner, using the generated set of compliance functions; and
estimate blood pressure of the subject, by the in-silico cardiovascular hemodynamic model via the one or more hardware processors, using the estimated cardiac parameters and the sequentially activated plurality of cardiac chambers to simulate the in-silico cardiovascular hemodynamic model in line with a human cardiovascular system of the subject, wherein the in-silico cardiovascular hemodynamic model is driven by the ECG signal associated with the subject.

4. The system as claimed in claim 3, wherein the one or more processors are configured to estimate the set of compliance parameters of the $j^{th}$ cardiac cycle as:

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, T^j = t^j,$$

wherein,

*Tl* is the activation time across a right atrium (*ra*),
time t is considered over a complete cardiac cycle,

$d_{la}$ is a time delay between activation of the *ra* and a left atrium *la,*

*d* is a time delay when activation of the plurality of cardiac chambers traverses from an atrium to ventricles therein,

$C_{ra}^{max,j}$ and $C_{ra}^{min,j}$ represent a maximum and a minimum value of *ra* compliance,

$C_i$ represents compliance functions to actuate a right ventricle *rv* and a left ventricle *lv,* and

$g_1 = 5, g_2 = g_3 = 2,$ and $g_4 = 1$ are set empirically.

5. One or more non-transitory machine-readable information storage mediums comprising one or more instructions which when executed by one or more hardware processors cause:

estimating, by an in-silico cardiovascular hemodynamic model via one or more hardware processors, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on:

(i) a set of PQRST amplitudes; and

(ii) time-instances, ( $\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$ ]; $j \in m$)

for a $j^{th}$ cardiac cycle ($\forall j \in m$) of the ECG signal,

wherein the continuous HR is estimated, when the ECG signal is missing or noisy, and is represented as:

$$HR(t) = \begin{cases} h_{ae}(end) + w(t) & during\ resting\ state \\ \left[1 - (t+1)e^{-\frac{t}{\tau_k}}\right] h_{ae}(0) + w(t) & during\ exercising\ state \end{cases}$$

where t is time considered over a complete cardiac cycle (T), $h_{ae}$ (*end*) is the HR at a last instance of capturing of the ECG signal in the resting state and $h_{ae}$ (0) is the HR at a first instance of the capturing of the ECG signal in the exercise state, wherein the subject is initially in the resting state and then performs exercise to reach the exercise state, $w(t) = \mathbb{N}(0, \sigma^2)$ is white-noise with zero-mean, variance of $\sigma^2 = 9.26,$ and $\tau_k = 50$ sec defines a time constant, and wherein the $\sigma^2,$ and the $\tau_k$ are learnt empirically through the ECG signal using linear regression;

generating, by the in-silico cardiovascular hemodynamic model via the one or more hardware processors, a set of compliance functions using the estimated cardiac parameters;

sequentially activating, by the in-silico cardiovascular hemodynamic model via the one or more hardware processors, a plurality of cardiac chambers of the in-silico cardiovascular hemodynamic model, in a synchronized manner, using the generated set of compliance functions; and

estimating blood pressure of the subject, by the in-silico cardiovascular hemodynamic model via the one or more hardware processors, using the estimated cardiac parameters and the sequentially activated plurality of cardiac chambers to simulate the in-silico cardiovascular hemodynamic model in line with a human cardiovascular system of the subject, wherein the in-silico cardiovascular hemodynamic model is driven by the ECG signal associated with the subject.

6. The one or more non-transitory machine-readable information storage mediums as claimed in claim 5, wherein the set of compliance parameters of the $j^{th}$ cardiac cycle is estimated as

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4}$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \quad T^j = t^j,$$

wherein,

*Tl* is the activation time across a right atrium (*ra*),

time *t* is considered over a complete cardiac cycle,

$d_{la}$ is a time delay between activation of the *ra* and a left atrium *la,*

d is a time delay when activation of the plurality of cardiac chambers traverses from an atrium to ventricles therein,

$C_{ra}^{max,j}$ and $C_{ra}^{min,j}$ represent a maximum and a minimum value of *ra* compliance,

$C_i$ represents compliance functions to actuate a right ventricle *rv* and a left ventricle *lv*, and

$g_1 = 5$, $g_2 = g_3 = 2$, and $g_4 = 1$ are set empirically.

**Patentansprüche**

1. Prozessorimplementiertes Verfahren (300), umfassend:

Schätzen, durch ein in-silico-kardiovaskuläres hämodynamisches Modell über einen oder mehrere Hardwareprozessoren, in jedem Herzzyklus eines Elektrokardiogramm(EKG)-Signals, von Herzparametern basierend auf einer Morphologie des EKG-Signals, das mit einem Subjekt assoziiert ist, wobei die Herzparameter eine kontinuierliche Herzrate (HR) und einen Satz von Compliance-Parametern beinhalten, und wobei das Schätzen des Satzes von Compliance-Parametern basiert auf:

(i) einem Satz von PQRST-Amplituden; und

(ii) Zeitpunkten, ( $\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$ ; $j \in m$) für einen $j^{ten}$ Herzzyklus ($\forall j \in m$) des EKG-Signals (302),

wobei die kontinuierliche HR geschätzt wird, wenn das EKG-Signal fehlt oder verrauscht ist, und dargestellt wird als:

$$HR(t) =$$

$$\begin{cases} h_{ae}(Ende) + w(t) & während\ Ruhezustand \\ \left[1 - (t+1)e^{-\frac{t}{\tau_k}}\right] h_{ae}(0) + w(t) & während\ Trainingszustand \end{cases}$$

wobei t die Zeit ist, die über einen vollständigen Herzzyklus (T) betrachtet wird, $h_{ae}$(*Ende*) die HR bei einem letzten Zeitpunkt des Erfassens des EKG-Signals im Ruhezustand ist und $h_{ae}$(0) die HR bei einem ersten Zeitpunkt des Erfassens des EKG-Signals im Trainingszustand ist, wobei das Subjekt anfänglich im Ruhezustand ist und dann eine Übung durchführt, um den Trainingszustand zu erreichen, $w(t) = \mathbb{N}(0, \sigma^2)$ weißes Rauschen mit einem Mittelwert von null, einer Varianz von $\sigma^2$ = 9,26 ist und $\tau_k$ = 50 Sekunden eine Zeitkonstante definiert, und wobei das $\sigma^2$ und das $\tau_k$ durch das EKG-Signal unter Verwendung einer linearen Regression empirisch gelernt werden;

Erzeugen, durch das in-silico-kardiovaskuläre hämodynamische Modell über den einen oder die mehreren Hardwareprozessoren, eines Satzes von Compliance-Funktionen unter Verwendung der geschätzten Herzparameter (304);

sequenzielles Aktivieren, durch das in-silico-kardiovaskuläre hämodynamische Modell über den einen oder die mehreren Hardwareprozessoren, einer Mehrzahl von Herzkammern des in-silicokardiovaskulären hämodynamischen Modells auf eine synchronisierte Weise unter Verwendung des erzeugten Satzes von Compliance-Funktionen (306); und

Schätzen des Blutdrucks des Subjekts, durch das in-silico-kardiovaskuläre hämodynamische Modell über den einen oder die mehreren Hardwareprozessoren, unter Verwendung der geschätzten Herzparameter und der sequenziell aktivierten Mehrzahl von Herzkammern, um das in-silico-kardiovaskuläre hämodynamische Modell in Übereinstimmung mit einem menschlichen kardiovaskulären System des Subjekts zu simulieren, wobei das in-silico-kardiovaskuläre hämodynamische Modell durch das EKG-Signal, das mit dem Subjekt assoziiert ist, angetrieben wird (308).

2. Prozessorimplementiertes Verfahren nach Anspruch 1, wobei der Satz von Compliance-Parametern des $j^{ten}$ Herzzyklus geschätzt wird als

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \ T^j = t^j,$$

wobei

$T_a^j$ die Aktivierungszeit über einen rechten Vorhof (*ra*) ist,
Zeit t über einen vollständigen Herzzyklus betrachtet wird,

$d_{la}$ eine Zeitverzögerung zwischen der Aktivierung des *ra* und eines linken Vorhofs *la* ist,

*d* eine Zeitverzögerung ist, wenn die Aktivierung der Mehrzahl von Herzkammern von einem Vorhof zu Ventrikeln darin verläuft,

$C_{ra}^{max,j}$ und $C_{ra}^{min,j}$ einen maximalen und einen minimalen Wert der *ra* Compliance darstellen,

$C_i$ Compliance-Funktionen darstellt, um einen rechten Ventrikel *rv* und einen linken Ventrikel *lv* zu betätigen, und

$g_1 = 5$, $g_2 = g_3 = 2$ und $g_4 = 1$ empirisch eingestellt werden.

3. System (100), umfassend:

einen Speicher (102), der Anweisungen in einem in-silicokardiovaskulären hämodynamischen Modell speichert;
eine oder mehrere Kommunikationsschnittstellen (106); und
einen oder mehrere Hardwareprozessoren (104), die über die eine oder die mehreren Kommunikationsschnittstellen (106) mit dem Speicher (102) gekoppelt sind, wobei der eine oder die mehreren Hardwareprozessoren (104) durch die Anweisungen konfiguriert sind zum:

Schätzen, in jedem Herzzyklus eines Elektrokardiogramm(EKG)-Signals, von Herzparametern basierend auf einer Morphologie des EKG-Signals, das mit einem Subjekt assoziiert ist, wobei die Herzparameter eine kontinuierliche Herzrate (HR) und einen Satz von Compliance-Parametern beinhalten, und wobei das Schätzen des Satzes von Compliance-Parametern basiert auf:

(i) einem Satz von PQRST-Amplituden; und

(ii) Zeitpunkten, ( $\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$ ; $j \in$ m) für einen $j^{ten}$ Herzzyklus ($\forall j \in m$) des EKG-Signals,

wobei die kontinuierliche HR geschätzt wird, wenn das EKG-Signal fehlt oder verrauscht ist, und dargestellt wird als:

$$HR(t) =$$

$$\begin{cases} h_{ae}(Ende) + w(t) & während\ Ruhezustand \\ \left[1 - (t+1)e^{-\frac{t}{\tau_k}}\right]h_{ae}(0) + w(t) & während\ Trainingszustand \end{cases}$$

wobei t die Zeit ist, die über einen vollständigen Herzzyklus (T) betrachtet wird, $h_{ae}$(*Ende*) die HR bei einem letzten Zeitpunkt des Erfassens des EKG-Signals im Ruhezustand ist und $h_{ae}$(0) die HR bei einem ersten Zeitpunkt des Erfassens des EKG-Signals im Trainingszustand ist, wobei das Subjekt anfänglich im Ruhezustand ist und dann eine Übung durchführt, um den Trainingszustand zu erreichen, $w(t) = \mathbb{N}(0, \sigma^2)$ weißes Rauschen mit einem Mittelwert von null, einer Varianz von $\sigma^2 = 9{,}26$ ist und $\tau_k = 50$ Sekunden eine Zeitkonstante definiert, und wobei das $\sigma^2$ und das $\tau_k$ durch das EKG-Signal unter Verwendung einer linearen Regression empirisch gelernt werden;
Erzeugen, eines Satzes von Compliance-Funktionen unter Verwendung der geschätzten Herzparameter;
sequenzielles Aktivieren, einer Mehrzahl von Herzkammern des in-silico-kardiovaskulären hämodynamischen Modells auf eine synchronisierte Weise unter Verwendung des erzeugten Satzes von Compliance-Funktionen; und
Schätzen des Blutdrucks des Subjekts, durch das in-silico-kardiovaskuläre hämodynamische Modell über den einen oder die mehreren Hardwareprozessoren, unter Verwendung der geschätzten Herzparameter und der sequenziell aktivierten Mehrzahl von Herzkammern, um das in-silico-kardiovaskuläre hämodynamische Modell in Übereinstimmung mit einem menschlichen kardiovaskulären System des Subjekts zu simulieren, wobei das in-silico-kardiovaskuläre hämodynamische Modell durch das EKG-Signal, das mit dem Subjekt assoziiert ist, angetrieben wird.

4. System nach Anspruch 3, wobei der eine oder die mehreren Prozessoren konfiguriert sind, um den Satz von Compliance-Parametern des $j^{ten}$ Herzzyklus zu schätzen als:

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, T^j = t^j,$$

wobei

$T_a^j$ die Aktivierungszeit über einen rechten Vorhof (ra) ist,
Zeit t über einen vollständigen Herzzyklus betrachtet wird,
$d_{la}$ eine Zeitverzögerung zwischen der Aktivierung des ra und eines linken Vorhofs la ist,
d eine Zeitverzögerung ist, wenn die Aktivierung der Mehrzahl von Herzkammern von einem Vorhof zu Ventrikeln darin verläuft,
$C_{ra}^{max,j}$ und $C_{ra}^{min,j}$ einen maximalen und einen minimalen Wert der ra Compliance darstellen,
$C_i$ Compliance-Funktionen darstellt, um einen rechten Ventrikel rv und einen linken Ventrikel lv zu betätigen, und
$g_1 = 5$, $g_2 = g_3 = 2$ und $g_4 = 1$ empirisch eingestellt werden.

5. Ein oder mehrere nichtflüchtige maschinenlesbare Informationsspeichermedien, umfassend eine oder mehrere Anweisungen, die, wenn sie von einem oder mehreren Hardwareprozessoren ausgeführt werden, Folgendes bewirken:

Schätzen, durch ein in-silico-kardiovaskuläres hämodynamisches Modell über einen oder mehrere Hardwareprozessoren, in jedem Herzzyklus eines Elektrokardiogramm(EKG)-Signals, von Herzparametern basierend auf einer Morphologie des EKG-Signals, das mit einem Subjekt assoziiert ist, wobei die Herzparameter eine kontinuierliche Herzrate (HR) und einen Satz von Compliance-Parametern beinhalten, und wobei das Schätzen des Satzes von Compliance-Parametern basiert auf:

(i) einem Satz von PQRST-Amplituden; und

(ii) Zeitpunkten, ( $\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]$ ; $j \in m$) für einen $j^{ten}$ Herzzyklus ($\forall j \in m$) des EKG-Signals,

wobei die kontinuierliche HR geschätzt wird, wenn das EKG-Signal fehlt oder verrauscht ist, und dargestellt wird als:

$$HR(t) =$$

$$\begin{cases} h_{ae}(Ende) + w(t) & während\ Ruhezustand \\ \left[1 - (t+1)e^{-\frac{t}{\tau_k}}\right]h_{ae}(0) + w(t) & während\ Trainingszustand \end{cases}$$

wobei t die Zeit ist, die über einen vollständigen Herzzyklus (T) betrachtet wird, $h_{ae}(Ende)$ die HR bei einem letzten Zeitpunkt des Erfassens des EKG-Signals im Ruhezustand ist und $h_{ae}(0)$ die HR bei einem ersten Zeitpunkt des Erfassens des EKG-Signals im Trainingszustand ist, wobei das Subjekt anfänglich im Ruhezustand ist und dann eine Übung durchführt, um den Trainingszustand zu erreichen, $w(t) = \mathbb{N}(0, \sigma^2)$ weißes Rauschen mit einem Mittelwert von null, einer Varianz von $\sigma^2 = 9{,}26$ ist und $\tau_k = 50$ Sekunden eine Zeitkonstante definiert, und wobei das $\sigma^2$ und das $\tau_k$ durch das EKG-Signal unter Verwendung einer linearen Regression empirisch gelernt werden;

Erzeugen, durch das in-silico-kardiovaskuläre hämodynamische Modell über den einen oder die mehreren Hardwareprozessoren, eines Satzes von Compliance-Funktionen unter Verwendung der geschätzten Herzparameter;

sequenzielles Aktivieren, durch das in-silico-kardiovaskuläre hämodynamische Modell über den einen oder die mehreren Hardwareprozessoren, einer Mehrzahl von Herzkammern des in-silicokardiovaskulären hämodynamischen Modells auf eine synchronisierte Weise unter Verwendung des erzeugten Satzes von Compliance-Funktionen; und

Schätzen des Blutdrucks des Subjekts, durch das in-silico-kardiovaskuläre hämodynamische Modell über den einen oder die mehreren Hardwareprozessoren, unter Verwendung der geschätzten Herzparameter und der sequenziell aktivierten Mehrzahl von Herzkammern, um das in-silico-kardiovaskuläre hämodynamische Modell in Übereinstimmung mit einem menschlichen kardiovaskulären System des Subjekts zu simulieren, wobei das in-silico-kardiovaskuläre hämodynamische Modell durch das EKG-Signal, das mit dem Subjekt assoziiert ist, angetrieben wird.

6. Ein oder mehrere nichtflüchtige maschinenlesbare Informationsspeichermedien nach Anspruch 5, wobei der Satz von Compliance-Parametern des $j^{ten}$ Herzzyklus geschätzt wird als

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4}$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \ T^j = t^j,$$

wobei

$T_a^j$ die Aktivierungszeit über einen rechten Vorhof (*ra*) ist,

Zeit *t* über einen vollständigen Herzzyklus betrachtet wird,

$d_{la}$ eine Zeitverzögerung zwischen der Aktivierung des *ra* und eines linken Vorhofs *la* ist,

d eine Zeitverzögerung ist, wenn die Aktivierung der Mehrzahl von Herzkammern von einem Vorhof zu Ventrikeln darin verläuft,

$C_{ra}^{max,j}$ und $C_{ra}^{min,j}$ einen maximalen und einen minimalen Wert der *ra* Compliance darstellen,

$C_i$ Compliance-Funktionen darstellt, um einen rechten Ventrikel *rv* und einen linken Ventrikel *lv* zu betätigen, und

$g_1$ = 5, $g_2$ = $g_3$ = 2 und $g_4$ = 1 empirisch eingestellt werden.

**Revendications**

1. Procédé mis en oeuvre par processeur (300) comprenant :

l'estimation, par un modèle hémodynamique cardiovasculaire in silico via un ou plusieurs processeurs matériels pour chaque cycle cardiaque d'un signal d'électrocardiogramme (ECG), sur la base de la morphologie du signal ECG associé à un sujet, dans lequel les paramètres cardiaques incluent une fréquence cardiaque (HR) continue et un ensemble de paramètres de compliance, et dans lequel l'estimation de l'ensemble de paramètres de compliance est basée sur :

(i) un ensemble d'amplitudes PQRST ; et
(ii) des instances temporelles,

$$\left( \left[ \left( a_p^j, t_p^j \right), \left( a_q^j, t_q^j \right), \left( a_r^j, t_r^j \right), \left( a_s^j, t_s^j \right), \left( a_t^j, t_t^j \right) \right] ; j \in m \right)$$

pour un cycle cardiaque $j^{th}$ ($\forall j \in m$) du signal ECG (302),
dans lequel la HR continue est estimée, lorsque le signal ECG est manquant ou bruyant, et est représentée comme :

$$HR(t) = \begin{cases} h_{ae}(fin) + w(t) & en \ état \ de \ repos \\ \left[ 1 - (t+1)e^{-\frac{t}{\tau_k}} \right] h_{ae}(0) + w(t) & en \ état \ d'exercice \end{cases}$$

où t est le temps considéré sur un cycle cardiaque complet (T), $h_{ae}(end)$ est la HR à une dernière instance de capture du signal ECG dans l'état de repos et $h_{ae}(0)$ est la HR à une première instance de la capture du signal ECG dans l'état d'exercice, dans lequel le sujet est initialement en état de repos et effectue ensuite un exercice pour atteindre l'état d'exercice, $w(t) = \mathbb{N}(0, \sigma^2)$ est un bruit blanc avec une variance moyenne nulle de $\sigma^2$ = 9.26, et $\tau_k$ = 50 définit une constante de temps, et dans lequel les valeurs $\sigma^2$, et les $\tau_k$ sont apprises empiriquement à travers le signal ECG en utilisant une régression linéaire ;

la génération, par le modèle hémodynamique cardiovasculaire in silico via le ou les processeurs matériels, d'un ensemble de fonctions de compliance en utilisant les paramètres cardiaques estimés (304) ;

l'activation séquentielle, par le modèle hémodynamique cardiovasculaire in silico via le ou les processeurs matériels, d'une pluralité de cavités cardiaques du modèle hémodynamique cardiovasculaire in silico, d'une manière synchronisée, en utilisant l'ensemble généré de fonctions de compliance (306) ; et
l'estimation de la pression artérielle du sujet, par le modèle hémodynamique cardiovasculaire in silico via le ou les processeurs matériels, en utilisant les paramètres cardiaques estimés et la pluralité de cavités cardiaques activées séquentiellement pour simuler le modèle hémodynamique cardiovasculaire in silico en adéquation avec un système cardiovasculaire humain du sujet, dans lequel le modèle hémodynamique cardiovasculaire in silico est piloté par le signal ECG associé au sujet (308).

2. Procédé mis en oeuvre par processeur selon la revendication 1, dans lequel l'ensemble de paramètres de compliance du cycle cardiaque $j^{th}$ est estimé comme

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \ T^j = t^j,$$

dans lequel,

$T_a^j$ est le temps d'activation à travers une oreillette droite (*ra*),

le temps t est considéré sur un cycle cardiaque complet,

$d_{la}$ est un retard temporel entre l'activation de l'oreillette droite *ra* et d'une oreillette gauche *la,*

*d* est un retard temporel lorsque l'activation de la pluralité de cavités cardiaques passe d'une oreillette aux ventricules correspondants,

$C_{ra}^{max,j}$ et $C_{ra}^{min,j}$ représentent respectivement une valeur maximale et une valeur minimale de compliance de l'oreillette droite *ra,*

$C_i$ représente des fonctions de compliance pour actionner un ventricule droit *rv* et un ventricule gauche *lv,* et

$g_1 = 5$, $g_2 = g_3 = 2$, et $g_4 = 1$ sont définis empiriquement.

3. Système (100) comprenant :

une mémoire (102) stockant des instructions dans un modèle hémodynamique cardiovasculaire in silico ;
une ou plusieurs interfaces de communication (106) ; et
un ou plusieurs processeurs matériels (104) couplés à la mémoire (102) via la ou les interfaces de communication (106), dans lequel le ou les processeurs matériels (104) sont configurés par les instructions pour :

l'estimation pour chaque cycle cardiaque d'un signal d'électrocardiogramme (ECG), sur la base de la morphologie du signal ECG associé à un sujet, dans lequel les paramètres cardiaques incluent une fréquence cardiaque (HR) continue et un ensemble de paramètres de compliance, et dans lequel l'estimation de l'ensemble de paramètres de compliance est basée sur :

(i) un ensemble d'amplitudes PQRST ; et
(ii) des instances temporelles,

$$\left(\left[\left(a_p^j, t_p^j\right), \left(a_q^j, t_q^j\right), \left(a_r^j, t_r^j\right), \left(a_s^j, t_s^j\right), \left(a_t^j, t_t^j\right)\right]; j \in m\right)$$

pour un cycle cardiaque $j^{th}$ ( $\forall j \in m$) du signal ECG (302),
dans lequel la HR continue est estimée, lorsque le signal ECG est manquant ou bruyant, et est représentée comme :

$$HR(t) = \begin{cases} h_{ae}(fin) + w(t) & en\ état\ de\ repos \\ \left[1 - (t+1)e^{-\frac{t}{\tau_k}}\right]h_{ae}(0) + w(t) & en\ état\ d'exercice \end{cases}$$

où t est le temps considéré sur un cycle cardiaque complet (T), $h_{ae}(end)$ est la HR à une dernière instance de capture du signal ECG dans l'état de repos et $h_{ae}(0)$ est la HR à une première instance de la capture du signal ECG dans l'état d'exercice, dans lequel le sujet est initialement en état de repos et effectue ensuite un exercice pour atteindre l'état d'exercice, $w(t) = \mathbb{N}(0, \sigma^2)$ est un bruit blanc avec une variance moyenne nulle de $\sigma^2$ = 9.26, et $\tau_k$ = 50 définit une constante de temps, et dans lequel les valeurs $\sigma^2$, et les $\tau_k$ sont apprises empiriquement à travers le signal ECG en utilisant une régression linéaire ;
la génération d'un ensemble de fonctions de compliance en utilisant les paramètres cardiaques estimés (304) ;
l'activation séquentielle d'une pluralité de cavités cardiaques du modèle hémodynamique cardiovasculaire in silico, d'une manière synchronisée, en utilisant l'ensemble généré de fonctions de compliance (306) ; et
l'estimation de la pression artérielle du sujet, par le modèle hémodynamique cardiovasculaire in silico via le ou les processeurs matériels, en utilisant les paramètres cardiaques estimés et la pluralité de cavités cardiaques activées séquentiellement pour simuler le modèle hémodynamique cardiovasculaire in silico en adéquation avec un système cardiovasculaire humain du sujet, dans lequel le modèle hémodynamique cardiovasculaire in silico est piloté par le signal ECG associé au sujet (308).

4. Système selon la revendication 3, dans lequel le un ou les processeurs sont configurés pour estimer l'ensemble de paramètres de compliance du cycle cardiaque $j^{th}$ comme :

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4},$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, T^j = t^j,$$

dans lequel,

$T_a^j$ est le temps d'activation à travers une oreillette droite (*ra*),

le temps *t* est considéré sur un cycle cardiaque complet,

*d$_{la}$* est un retard temporel entre l'activation de l'oreillette droite *ra* et d'une oreillette gauche *la*,

*d* est un retard temporel lorsque l'activation de la pluralité de cavités cardiaques passe d'une oreillette aux ventricules correspondants,

$C_{ra}^{max,j}$ et $C_{ra}^{min,j}$ représentent respectivement une valeur maximale et une valeur minimale de compliance de l'oreillette droite *ra*,

*C$_i$* représente des fonctions de compliance pour actionner un ventricule droit *rv* et un ventricule gauche *lv*, et

$g_1 = 5$, $g_2 = g_3 = 2$, et $g_4 = 1$ sont définis empiriquement.

5. Un ou plusieurs supports de stockage d'informations lisibles par machine non transitoires comprenant une ou plusieurs instructions qui, lorsqu'elles sont exécutées par un ou plusieurs processeurs matériels, entraînent :

l'estimation, par un modèle hémodynamique cardiovasculaire in silico via un ou plusieurs processeurs matériels pour chaque cycle cardiaque d'un signal d'électrocardiogramme (ECG), sur la base de la morphologie du signal ECG associé à un sujet, dans lequel les paramètres cardiaques incluent une fréquence cardiaque (HR) continue et un ensemble de paramètres de compliance, et dans lequel l'estimation de l'ensemble de paramètres de compliance est basée sur :

(i) un ensemble d'amplitudes PQRST ; et
(ii) des instances temporelles,

$$\left( \left[ \left( a_p^j, t_p^j \right), \left( a_q^j, t_q^j \right), \left( a_r^j, t_r^j \right), \left( a_s^j, t_s^j \right), \left( a_t^j, t_t^j \right) \right]; j \in m \right)$$

pour un cycle cardiaque *j$^{th}$* ( $\forall j \in m$) du signal ECG (302),

dans lequel la HR continue est estimée, lorsque le signal ECG est manquant ou bruyant, et est représentée comme :

$$HR(t) = \begin{cases} h_{ae}(fin) + w(t) & en\ état\ de\ repos \\ \left[ 1 - (t+1)e^{-\frac{t}{\tau_k}} \right] h_{ae}(0) + w(t) & en\ état\ d'exercice \end{cases}$$

où t est le temps considéré sur un cycle cardiaque complet (T), *h$_{ae}$*(*end*) est la HR à une dernière instance de capture du signal ECG dans l'état de repos et *h$_{ae}$*(0) est la HR à une première instance de la capture du signal ECG dans l'état d'exercice, dans lequel le sujet est initialement en état de repos et effectue ensuite un exercice pour atteindre l'état d'exercice, $w(t) = \mathbb{N}(0, \sigma^2)$ est un bruit blanc avec une variance moyenne nulle de $\sigma^2 = 9.26$, et $\tau_k = 50$ définit une constante de temps, et dans lequel les valeurs $\sigma^2$, et les $\tau_k$ sont apprises empiriquement à travers le signal ECG en utilisant une régression linéaire ;

la génération, par le modèle hémodynamique cardiovasculaire in silico via le ou les processeurs matériels, d'un ensemble de fonctions de compliance en utilisant les paramètres cardiaques estimés;

l'activation séquentielle, par le modèle hémodynamique cardiovasculaire in silico via le ou les processeurs matériels, d'une pluralité de cavités cardiaques du modèle hémodynamique cardiovasculaire in silico, d'une manière synchronisée, en utilisant l'ensemble généré de fonctions de compliance ; et

l'estimation de la pression artérielle du sujet, par le modèle hémodynamique cardiovasculaire in silico via le ou les processeurs matériels, en utilisant les paramètres cardiaques estimés et la pluralité de cavités cardiaques activées séquentiellement pour simuler le modèle hémodynamique cardiovasculaire in silico en adéquation avec un système cardiovasculaire humain du sujet, dans lequel le modèle hémodynamique cardiovasculaire in silico est piloté par le signal ECG associé au sujet.

6. Un ou plusieurs supports de stockage d'informations lisibles par machine non transitoires selon la revendication 5, dans lequel l'ensemble de paramètres de compliance du cycle cardiaque $j^{th}$ est estimé comme

$$T_a^j = t_p^j,$$

$$d_{la}^j = \frac{g_1 t_p^j + g_2 t_q^j}{g_1 + g_2},$$

$$T_1^j = \frac{g_3 t_r^j + g_4 t_t^j}{g_3 + g_4}$$

$$T_2^j = t_t^j,$$

$$d^j = t_q^j - t_p^j,$$

$$C_{ra}^{max,j} = C_{la}^{max,j} = \frac{a_p^j}{a_q^j},$$

$$C_{ra}^{min,j} = C_{la}^{min,j} = 0.2,$$

$$C_i = \frac{a_r^j}{a_q^j}, \; T^j = t^j,$$

dans lequel,

$T_a^j$ est le temps d'activation à travers une oreillette droite (*ra*),

le temps *t* est considéré sur un cycle cardiaque complet,

$d_{la}$ est un retard temporel entre l'activation de l'oreillette droite *ra* et d'une oreillette gauche *la*,

d est un retard temporel lorsque l'activation de la pluralité de cavités cardiaques passe d'une oreillette aux ventricules correspondants,

$C_{ra}^{max,j}$ et $C_{ra}^{min,j}$ représentent respectivement une valeur maximale et une valeur minimale de compliance de l'oreillette droite *ra*,

$C_i$ représente des fonctions de compliance pour actionner un ventricule droit *rv* et un ventricule gauche *lv*, et

$g_1 = 5$, $g_2 = g_3 = 2$, et $g_4 = 1$ sont définis empiriquement.

SYSTEM **100**

MEMORY **102**

IN-SILICO
CARDIOVASCULAR
HEMODYNAMIC MODEL

HARDWARE PROCESSOR(S)
**104**

I/O INTERFACE(S) **106**

FIG.1

In-silico
cardiovascular
hemodynamic
model

Captured ECG signal
of a subject → Compliance
parameters → Blood pressure

Continuous
Heart Rate

FIG.2

FIG.3 — 300

estimating, by an in-silico cardiovascular hemodynamic model via one or more hardware processors, in each cardiac cycle of an electrocardiogram (ECG) signal, cardiac parameters based on morphology of the ECG signal associated with a subject, wherein the cardiac parameters include a continuous heart rate (HR) and a set of compliance parameters, and wherein estimating the set of compliance parameters is based on (i) a set of PQRST amplitudes; and (ii) time-instances, for a jth cardiac cycle of the ECG signal — 302

generating, by the in-silico cardiovascular hemodynamic model via the one or more hardware processors, a set of compliance functions using the estimated cardiac parameters — 304

sequentially activating, by the in-silico cardiovascular hemodynamic model via the one or more hardware processors, a plurality of cardiac chambers, in a synchronized manner, using the generated set of compliance functions — 306

estimating blood pressure of the subject, by the in-silico cardiovascular hemodynamic model via the one or more hardware processors, wherein the in-silico cardiovascular hemodynamic model is driven by the ECG signal associated with the subject — 308

FIG.4A

(PRIOR ART)

FIG.4B

FIG.5

FIG.6A

(PRIOR ART)

FIG.6B

FIG.7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202221033450

- WO 202121010972 A

**Non-patent literature cited in the description**

- **ROY DIBYENDU et al.** *Wearable sensor driven Cardiac model to derive hemodynamic insights during exercise*
- **SILVA LUCAS RAPHAEL BENTO et al.** *Exponential model for analysis of heart rate responses and autonomic cardiac modulation during different intensities of physical exercise*

- **A.ESMAILI.** Nonlinear cuffless blood pressure estimation of healthy subjects using pulse transit time and arrival time. *IEEE Transactions on Instrumentation and Measurement*